# EUROPEAN PATENT APPLICATION

(11) **EP 2 517 670 A1**
(43) Date of publication of application: **31.10.2012**
(21) Application number: 12165058.4
(22) Date of filing: 20.04.2012
(51) Int. Cl.: A61F 2/00

(54) **Prosthesis, in particular for urogynecological and andrological treatments**

(30) Priority: 26.04.2011 IT TO20110038 U
(71) Applicant: Dipro Medical Devices S.r.l., San Mauro Torinese (IT)
(72) Inventor: Buemi, Fabrizio, 10020 Pecetto Torinese (IT); Buemi, Cristina, 10090 San Raffaele Cimena (IT)
(74) Representative: Jorio, Paolo

(57) **Abstract**

A prosthesis (1;1a;1b;1c;1d;1e), in particular for urogynecological and andrological treatments, has an intermediate portion (2;2a;2b;2c;2d;2e) having, in use, a supporting function for a part of the body; at least two lateral arms (3;3a;3b;3c;3d;3e) project in a cantilever fashion from the intermediate portion and have respective external end portions (4;4a;4b;4c;4d;4e) that define connecting areas and made in the guise of a mesh of non-resorbable biocompatible polymer material; on the other hand, the intermediate portion comprises a film made of non-resorbable biocompatible polymer material fixed to the mesh.

## Description

The present innovation relates to a prosthesis, in particular for urogynecological and andrological treatments.

Prostheses are known which have been designed with the intention of taking action in the case of stress urinary incontinence (a condition which affects both women and men), in the case of prolapse of the vaginal wall (a possible consequence of childbirth or aging in women), and in the case of fecal incontinence. All these conditions, although not being directly life-threatening, often have serious psychological consequences on those affected by them. Prior art prostheses are applied surgically and are constituted entirely by a mesh, comprising an intermediate portion, which is placed in a position such as to perform a supporting and/or reinforcing function of the tissues, and at least one pair of lateral arms which, applied using the "tension free" technique, remain in a fixed position without any supplementary sutures.

The prior art prostheses type described above are not satisfactory, as in the majority of cases they cause some problems, in particular:
- pain and discomfort, especially during sexual activity;
- inflammation;
- internal lacerations and hemorrhaging;
- infections;
- recurrence of the problem.

The most common cause of these problems is inevitable rubbing of the mesh on the tissues against which the mesh is placed in contact, during normal movements performed in everyday life. To attempt to solve at least some of these problems, often the person must have further surgical operations which, however, can also worsen the situation.

Moreover, the tissues supported and/or reinforced by the intermediate portion of the prosthesis, being able to grow into the pores of the mesh, can lead to the formation of painful and unnatural masses, which must also be removed surgically.

The object of the present innovation is to provide a prosthesis, in particular for urogynecological and andrological treatments, which allows the problems set forth above to be solved in a simple and inexpensive manner.

According to the present innovation a prosthesis is provided, in particular for urogynecological and andrological treatments, the prosthesis comprising:
- an intermediate portion defining a supporting area for a part of the body; and
- at least two lateral arms, which project in a cantilever fashion with respect to said intermediate portion and comprise respective external end portions defining connecting areas and made in the guise of a mesh of non-resorbable biocompatible polymer material;
**characterized in that** said intermediate portion comprises a film made of non-resorbable biocompatible polymer material, fixed to said mesh.

Preferably, both faces of said intermediate portion are smooth, and said intermediate portion is constituted solely by said film and is fixed to said mesh at one edge thereof.

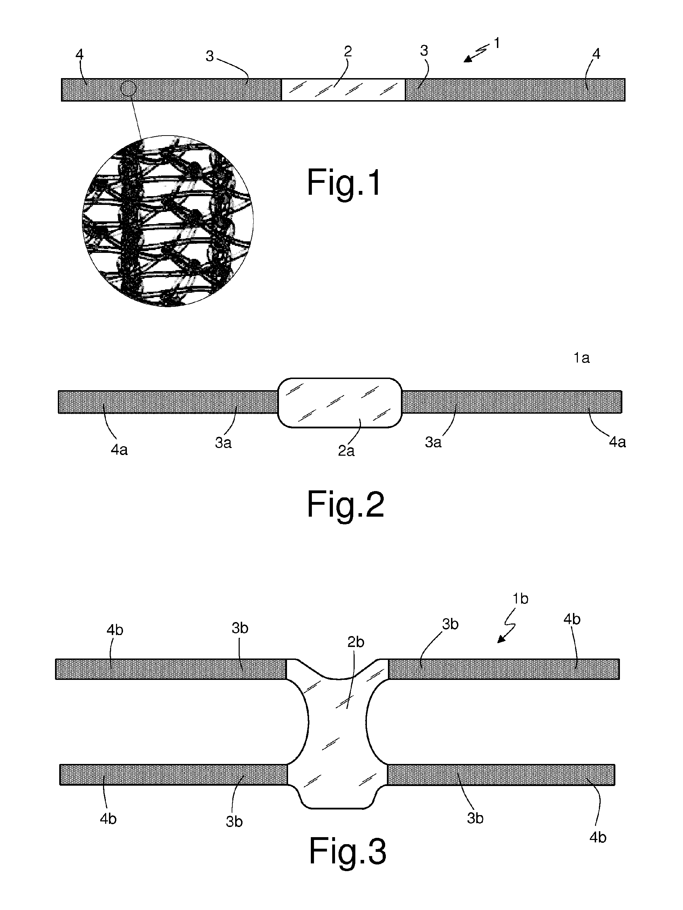

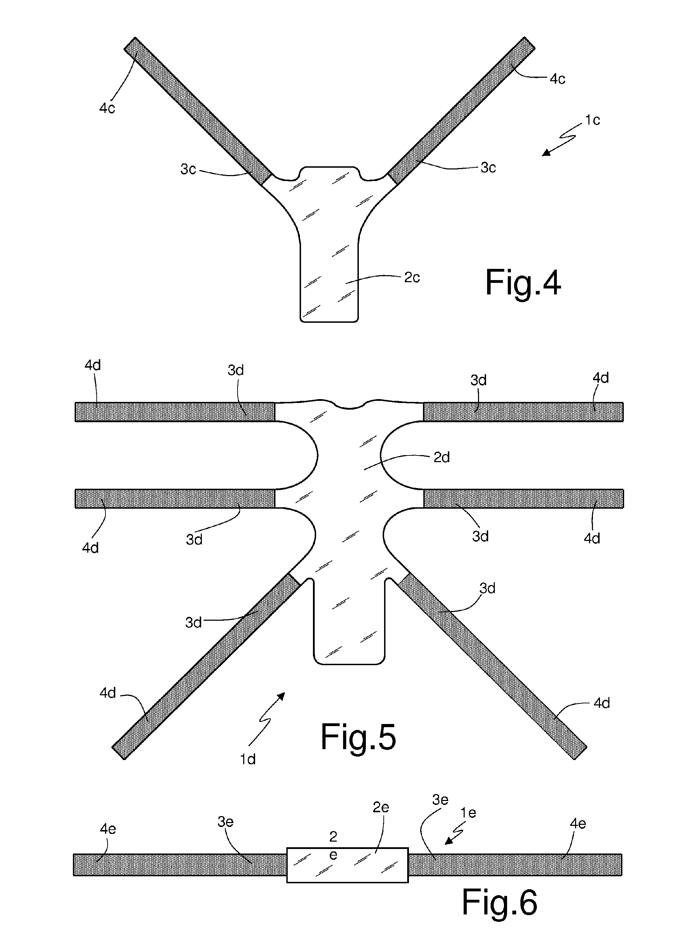

The innovation will now be described with reference to the accompanying drawings, which illustrate a non-limiting example of embodiment, and in which figures 1 to 6 respectively illustrate preferred embodiments of the prosthesis, in particular for urogynecological and andrological treatments, according to the present invention.

In Fig. 1, the number 1 indicates a prosthesis that can be used to treat female stress urinary incontinence.

The prosthesis 1 is substantially a band comprising an intermediate portion 2, which defines a supporting area adapted to support, in use, the urethra. The prosthesis 1 then comprises two arms 3, which are diametrically opposed and comprise, toward the outside, respective end portions 4 defining connecting areas adapted, in use, to be applied according to the "tension free" technique.

At least the portions 4 of the arms 3 are defined by respective mesh elements made of non-resorbable biocompatible polymer material, preferably polypropylene. On the other hand, the portion 2 is defined by a film made of non-resorbable biocompatible polymer material, preferably polypropylene, in particular of transparent type. In other words, the portion 2 is constituted by a sheet that has two substantially smooth faces and extends in a position such as to perform the supporting action. This action is no longer performed by a mesh element, as occurs in prior art prostheses, thereby minimizing the rubbing effects on an easily irritated area of the body.

The prosthesis 1 is constructed by alternating elements solely made of mesh with an element solely made of film. The mesh is fixed to one edge of the film, preferably by welding, for example heat or ultrasonic welding. In alternative to welding, gluing or stitching could be used.

In the embodiment of Fig. 1, the portion 2 has the same width as the arms 3.

Figs. 2 to 6 show other embodiments, the constituent parts of which are indicated, where possible, by the same reference numbers used in Fig. 1, but followed respectively by the reference letters "a" "b" "c" "d" "e".

The prosthesis 1a can be used for treating male incontinence. Unlike the prosthesis 1, the portion 2a made of film is wider with respect to the arms 3a made of mesh, as it must support, in use, the urethral bulb.

The prostheses 1b, 1c and 1d are adapted to solve a typically female problem, i.e. that of prolapse of the vaginal wall. In particular, the prostheses 1b, 1c and 1d can be used for the treatment, respectively, of:
- prolapse of the anterior vaginal wall, or anterior colpocele, or cystocele, and prolapse of the uterus or hysterocele;
- prolapse of the posterior vaginal wall, or posterior colpocele, or rectocele;
- total prolapse.

The prostheses 1b, 1c and 1d therefore differ in shape, but include the same constructional concept indicated above: the portions 4b, 4c, 4d are made of mesh, while the portions 2b, 2c, 2d are made of film, in order to minimize the risks of abrasion, inflammation and infection of the tissues with which it will be in contact after the surgical operation.

In particular, the prosthesis 1b has two pairs of arms 3b. The arms of each pair are mutually parallel and project from the portion 2b in the opposite direction with respect to the arms of the other pair. The two arms 3c of the prosthesis 1c mutually converge toward the portion 2c forming an angle of less than 180°. Finally, in the prosthesis 1d, being substantially the sum of the prosthesis 1b and of the prosthesis 1c, six arms 3d are provided, two of which converging toward the portion 2d, and four of which mutually parallel and arranged in pairs on the sides of the portion 2d.

Finally, the prosthesis 1e can be used for the treatment of fecal incontinence and has a shape analogous to that of the prosthesis 1b.

As mentioned above, with respect to prior art prostheses, the prostheses according to the present innovation comprise an intermediate or central portion made only of film, to avoid excoriations or inflammation in the more delicate areas with which the prostheses come into contact.

In fact, the film, is non-adhesive as it is defined by smooth surfaces, and therefore prevents or in any case greatly reduces the risks of the formation of adhesions with the muscle tissues of the body with which it will be placed in contact.

The mesh occupies at least the portions 4, 4a, 4b, 4c, 4d, 4e of the arms 3, 3a, 3b, 3c, 3d, 3e, or the connecting areas of the prosthesis. The mesh must preferably be selected in such a manner as to have sufficient tensile strength, good flexibility to facilitate insertion into the body during the surgical operation, but without excessive elongation and without excessive reduction in section and width when it is pulled towards the inside of the body, to connect the arms 3, 3a, 3b, 3c, 3d, 3e.

The mesh is macroporous (i.e. has pores with a mean diameter greater than 1 mm) and is defined by a weave of threads, each formed by a related polypropylene monofilament. The diameter of the threads is between 120 and 180 microns. Preferably, the weight of the mesh is between 40 and 220 g/m2.

In place of polypropylene, threads of different material can be used, of non-resorbable biocompatible type, such as: polyester, polyethylene terephthalate, polyamides, polyvinylidene fluoride, and mixtures thereof in different percentages.

Moreover, the material of the mesh could be covered by other materials/substances, for example by polyurethane and/or antimicrobial and antibacterial substances.

The film, with which the portions 2,2a,2b,2c,2d,2e (and optionally the internal ends of the arms) are made, is preferably a polypropylene homopolymer. However, the material to produce the film could be different, of non-resorbable biocompatible type, such as polytetrafluoroethylene, polysiloxanes, polyurethanes, precious metal derivatives, silicones, polyolefins, polyvinyldene fluoride, vinyldene fluoride, hexafluoropropylene, and composites thereof.

The film has a thickness preferably between 30 and 80 microns, is of single-layer type, and according to a variant not illustrated has a plurality of micro-perforations, for drainage of any fluids. The number and the diameter of these micro-perforations are relatively small, so that the non-adhesive properties of the faces of the film are not compromised. For example, the micro-perforations have a diameter in the order of one tenth of a millimeter, and are around ten in number.

From the above, it is evident that the prostheses 1,1a,1b,1c,1d,le of the present innovation limit abrasion with the tissues to be reinforced and/or supported after the surgical operation, as the supporting function is defined by a portion 2,2a,2b,2c,2d,2e made in the guise of a film having at least one smooth supporting surface.

Finally, from the above it is evident that modifications and variants can be made to the prostheses 1, 1a, 1b, 1c, 1d and 1e described and illustrated, without departing from the scope of protection of the present innovation, as defined in the appended claims.

In particular, the shape and dimensions of the lateral arms of the prostheses 1, 1a, 1b, 1c, 1d and 1e could differ from those illustrated by way of example.

## Claims

1. Prosthesis (1;1a;1b;1c;1d;1e), in particular for urogynecological and andrological treatments, the prosthesis comprising:
- an intermediate portion (2;2a;2b;2c;2d;2e) defining a supporting area for a part of the body; and
- at least two lateral arms (3;3a;3b;3c;3d;3e), which project in a cantilever fashion with respect to said intermediate portion and comprise respective external end portions (4;4a;4b;4c;4d;4e) defining connecting areas and made in the guise of a mesh of non-resorbable biocompatible polymer material;
**characterized in that** said intermediate portion (2;2a;2b;2c;2d;2e) comprises a film made of non-resorbable biocompatible polymer material, fixed to said mesh.

2. Prosthesis according to claim 1, **characterized in that** both the faces of said intermediate portion are smooth.

3. Prosthesis according to claim 2, **characterized in that** said intermediate portion (2;2a;2b;2c;2d;2e) is constituted solely by said film and is fixed to said mesh at one edge thereof.

4. Prosthesis according to claim 3, **characterized in that** the mesh is welded to said edge.

5. Prosthesis according to claim 3 or 4, **characterized in that** said film comprises a plurality of micro-perforations.

6. Prosthesis according to any one of the preceding claims, **characterized in that** said film has a thickness between 30 and 80 microns.

7. Prosthesis according to any one of the preceding claims, **characterized in that** said film is transparent.
